# EUROPÄISCHE PATENTSCHRIFT

(11) **EP 4 340 697 B1**
(45) Veröffentlichungstag und Bekanntmachung des Hinweises auf die Patenterteilung: **25.09.2024**
(21) Anmeldenummer: 22728776.0
(22) Anmeldetag: 10.05.2022
(51) Int. Cl.: A61B 1/12, B08B 9/02

(54) **MODUL ZUR BEAUFSCHLAGUNG VON SPÜLGÜTERN MIT EINEM MEDIUM**
MODULE FOR APPLYING A MEDIUM TO ITEMS TO BE WASHED
MODULE D'APPLICATION D'UN MILIEU À DES ARTICLES À LAVER

(30) Priorität: 21.05.2021 DE 102021113274
(43) Veröffentlichungstag der Anmeldung: 27.03.2024
(73) Patentinhaber: Miele & Cie. KG, 33332 Gütersloh (DE)
(72) Erfinder: WERNER, Alexander, 33619 Bielefeld (DE)
(86) Internationale Anmeldenummer: PCT/EP2022/062603
(87) Internationale Veröffentlichungsnummer: WO 2022/243108

(56) Entgegenhaltungen:
- EP-A1- 0 709 056
- EP-A1- 3 005 936
- EP-A2- 1 253 951
- DE-A1- 102018 121 450

## Beschreibung

Die Erfindung betrifft ein Modul zur Beaufschlagung von in einem Spülbehälter einer Reinigungseinrichtung angeordneten englumigen Kanälen, Leitungen, Rohren und/oder dgl. von Spülgütern, insbesondere Endoskopen, mit einem Medium, mit einer Mediumverteileinrichtung, die eine Vielzahl von eingangsseitigen Anschlüssen und ausgangsseitigen Anschlüssen aufweist, wobei die eingangsseitigen Anschlüsse mit den ausgangsseitigen Anschlüssen in strömungstechnischer Verbindung stehen.

Englumige Kanäle, Rohre, Leitungen und/oder dgl. im Sinne der Erfindung sind Kanäle, Rohre, Leitungen und/oder dgl. mit einem vergleichsweise kleinen inneren Querschnitt, d.h. einer vergleichsweise kleinen lichten Weite. Hierunter fallen insbesondere medizinische Instrumente, wie zum Beispiel minimalinvasive Instrumente, Dentalinstrumente, Kanülen, Katheter, Endoskope und/oder dgl.

Zur Reinigung englumiger Kanäle, Rohre, Leitungen und/oder dgl. ist es aus dem Stand der Technik bekannt, diese mit einer Reinigungsflüssigkeit zu beschicken. Infolge des Hindurchströmens der Reinigungsflüssigkeit durch die englumigen Kanäle, Rohre, Leitungen und/oder dgl. hindurch kommt es zu einem Abtrag der an der Innenoberfläche der englumigen Kanäle, Rohre, Leitungen und/oder dgl. unter Umständen anhaftenden Schmutz- und/oder Fremdpartikel.

Für eine derartige Einleitung von Reinigungsflüssigkeit in englumige Kanäle, Rohre, Leitungen und/oder dgl. ist aus dem Stand der Technik eine Verteilkammer bekannt, die einen eingangsseitigen Anschluss für eine Reinigungsflüssigkeit einerseits und eine Vielzahl von ausgangsseitigen Anschlüssen andererseits aufweist. Im bestimmungsgemäßen Verwendungsfall sind an die ausgangsseitigen Anschlüsse der Verteilkammer jeweils zu reinigende englumige Kanäle, Rohre, Leitungen und/oder dgl. angeschlossen.

Um eine weitere Verbesserung des Reinigungsergebnisses erzielen zu können, ist es aus dem Stand der Technik ferner bekannt, nicht nur Reinigungsflüssigkeit, sondern auch Druckluft durch die zu reinigenden englumigen Kanäle, Rohre, Leitungen und/oder dgl. zu führen.

Ein gattungsgemäßes Modul ist aus der DE 10 2018 121 450 A1 bekannt.

Das aus der DE 10 2018 121 450 A1 vorbekannte Modul verfügt über eine Mediumverteileinrichtung, die eine Mehrzahl von eingangsseitigen Anschlüssen einerseits und eine Mehrzahl von ausgangsseitigen Anschlüssen andererseits aufweist. Dabei stehen die eingangsseitigen Anschlüsse mit den ausgangsseitigen Anschlüssen in strömungstechnischer Verbindung, und zwar unter jeweiliger Zwischenschaltung eines Ventils, was eine wahlweise strömungstechnische Verbindung der ausgangsseitigen Anschlüsse mit den eingangsseitigen Anschlüssen ermöglicht. Als eingangsseitige Anschlüsse stehen ein Anschluss für eine Reinigungsflüssigkeit sowie zwei Druckluftanschlüsse zur Verfügung.

Obgleich sich das aus der DE 10 2018 121 450 A1 vorbekannte Modul im alltäglichen Praxiseinsatz bewährt hat, besteht Verbesserungsbedarf, insbesondere mit Blick auf eine verbesserte Energiebilanz bei der Durchführung einer bestimmungsgemäßen Reinigung und/oder Desinfektion. Es ist deshalb die **Aufgabe** der Erfindung, ein Modul der eingangs genannten Art konstruktiv dahingehend weiterzuentwickeln, dass ein energieeffizienterer Betrieb einer damit ausgerüsteten Reinigungseinrichtung ermöglicht ist.

Zur **Lösung** dieser Aufgabe wird mit der Erfindung ein Modul der eingangs genannten Art vorgeschlagen, das sich auszeichnet durch einen Trägerflansch, der einen Arbeitszylinder aufweist, wobei der Arbeitszylinder in strömungstechnischer Verbindung mit einem eingangsseitigen Anschluss steht, und wobei der Arbeitszylinder eine Kolbenstange bereitstellt, die endseitig einen mit einer Anschlusseinrichtung eines Spülgutträgers zusammenwirkenden Anker aufweist. Zudem wird mit der Erfindung eine mit einem solchen Modul ausgerüstete Reinigungseinrichtung vorgeschlagen.

Das erfindungsgemäße Modul verbindet in an sich bekannter Weise eingangsseitige Anschlüsse und ausgangsseitige Anschlüsse strömungstechnisch miteinander. Darüber hinaus stellt es einen Trägerflansch mit einem Arbeitszylinder bereit, der im bestimmungsgemäßen Verwendungsfall der Ankopplung an eine Anschlusseinrichtung eines Spülgutträgers dient. Dabei erfolgt im bestimmungsgemäßen Verwendungsfall eine Betätigung des Arbeitszylinders über ein Medium eines der eingangsseitigen Anschlüsse. Erfindungsgemäß ist deshalb vorgesehen, dass der Arbeitszylinder in strömungstechnischer Verbindung mit einem eingangsseitigen Anschluss steht.

Das erfindungsgemäße Modul ermöglicht mithin nicht nur eine Versorgung der zu reinigenden Spülgüter mit Reinigungsflüssigkeit und/oder Druckluft, sondern stellt auch eine Ankoppelfunktion bereit, so dass es im Unterschied zum Stand der Technik hierfür keiner zusätzlichen Bauteile oder Einrichtungen bedarf. Das erfindungsgemäße Modul stellt mithin eine Funktionseinheit bereit, die im bestimmungsgemäßen Verwendungsfall sowohl eine Mediumversorgung als auch eine fluidtechnische Ankopplung einer reinigungseinrichtungsseitigen Anschlusseinrichtung an die ausgangsseitigen Anschlüsse des Moduls bewerkstelligt.

Diese Funktionsintegration in nur eine Baugruppe bewirkt eine Vielzahl von Vorteilen. Im bestimmungsgemäßen Verwendungsfall ist eine kürzere Prozesszeit bei der Aufbereitung dadurch erreicht, dass die im Spülkreislauf befindliche Menge an Reinigungsflüssigkeit mangels zusätzlicher Verschlauchung reduziert ist, was mit kürzeren Aufheiz-, Wassereinlauf- und Wasserablaufvorgängen verbunden ist. Hieraus ergibt sich ein geringerer Energiebedarf für die Aufheizvorgänge der im Spülkreislauf befindlichen Reinigungsflüssigkeit. Zudem ist eine Montage deutlich schneller möglich, da viele Montageschritte im Unterschied zum Stand der Technik entfallen können, insbesondere solche, die zur Setzung von Schlauchverbindungen von Nöten sind. Denn Schlauchverbindungen zum strömungstechnischen Anschluss des erfindungsgemäßen Moduls an eine Reinigungseinrichtung bedarf es aufgrund der erfindungsgemäßen Ausgestaltung nicht mehr.

Vorzugsweise wird der Trägerflansch durch ein additives Fertigungsverfahren, ein spanendes Fertigungsverfahren oder Urformen hergestellt. Insbesondere kann der Trägerflansch durch ein 3D-Druckverfahren hergestellt sein, wobei speziell ein selektives Laserschmelzen von Metall, insbesondere Edelstahl, bevorzugt ist.

Der Arbeitszylinder des erfindungsgemäßen Moduls verfügt über eine Kolbenstange. Diese weist endseitig einen Anker auf. Dieser wirkt wiederum mit einer Anschlusseinrichtung eines Spülgutträgers zusammen, so dass bei einer Verfahrbewegung des Arbeitszylinders entweder ein Ankoppeln oder ein Abkoppeln stattfindet. Für einen Betrieb des Arbeitszylinders bedarf es in vorteilhafter Weise keines separat ausgebildeten Antriebs, da erfindungsgemäß vorgesehen ist, dass der Arbeitszylinder in strömungstechnischer Verbindung mit einem eingangsseitigen Anschluss steht. Dieser eingangsseitige Anschluss kann mithin zum einen dazu dienen, einen oder mehrere ausgangsseitige Anschlüsse mit einem Medium zu bedienen, zum anderen aber auch dafür, um einen bestimmungsgemäßen Betrieb des Arbeitszylinders zu bewirken. In vorteilhafter Weise wird so eine insgesamt kompakte Bauform des Moduls erreicht, das ohne weitere Bauteile und/oder Antriebe dazu ausgerüstet ist, in bestimmungsgemäßer Weise sowohl eine Mediumbeschickung der ausgangsseitigen Anschlüsse zu bewerkstelligen, als auch hinsichtlich einer reinigungseinrichtungsseitigen Ankoppeleinrichtung ein Ankoppeln bzw. Abkoppeln zu ermöglichen.

Insgesamt ist so ein Modul geschaffen, das eine vereinfachte Montage ermöglicht, kürzere Prozesszeiten gestattet und darüber hinaus einen energieeffizienteren Betrieb sicherstellt.

Gemäß einem weiteren Merkmal der Erfindung ist vorgesehen, dass der Arbeitszylinder ein pneumatischer Zylinder ist. Es kommt mithin ein druckluftbetriebener Zylinder zum Einsatz, der an einen eingangsseitigen Anschluss für Druckluft angeschlossen ist. Im Bedarfsfall kann so über den eingangsseitigen Anschluss eine Druckluftbeschickung des Arbeitszylinders stattfinden.

Gemäß einem weiteren Merkmal der Erfindung ist der pneumatische Zylinder ein Druckzylinder. Dabei erfolgt im bestimmungsgemäßen Verwendungsfall eine Druckluftbeaufschlagung des Zylinders derart, dass die Kolbenstange zwecks Ankopplung an die damit zusammenwirkende Anschlusseinrichtung von der Anschlusseinrichtung wegverfährt, der von der Kolbenstange bereitgestellte Anker mithin als Zuganker wirkt. Dies stellt eine sichere Verbindung von Anschlusseinrichtung und erfindungsgemäßem Modul sicher.

Gemäß einem weiteren Merkmal der Erfindung ist der Druckzylinder ein doppelwirkender Druckzylinder. Der Druckzylinder verfügt mithin über zwei Druckluftanschlüsse, so dass bei einer Beschickung über den einen Anschluss die Kolbenstange in die eine Richtung verfährt, wohingegen bei einer Beschickung des Zylinders über den anderen Anschluss die Kolbenstange in die entgegengesetzte Richtung verfährt. Unter Drucklufteinwirkung kann mithin sowohl ein Ankoppeln als auch ein Abkoppeln stattfinden. Es ist so ein verschleißarmer und damit dauerhaft sicherer Betrieb gewährleistet.

Gemäß einem weiteren Merkmal der Erfindung ist vorgesehen, dass ein erster eingangsseitiger Anschluss ein Anschluss für eine Reinigungsflüssigkeit ist. Über diesen Anschluss wird im bestimmungsgemäßen Verwendungsfall Reinigungsflüssigkeit, d.h. Spülflotte gefördert und einem oder mehreren ausgangsseitigen Anschlüssen zugeführt.

Gemäß einem weiteren Merkmal der Erfindung ist vorgesehen, dass ein zweiter eingangsseitiger Anschluss ein Anschluss für Druckluft eines ersten Druckniveaus von vorzugsweise unter 2 bar ist. Die Druckluft dieses Druckniveaus dient insbesondere dazu, wahlweise in Wechselwirkung mit der Reinigungsflüssigkeit durch die zu reinigenden englumigen Kanäle, Rohre, Leitungen und/oder dgl. hindurchgeführt zu werden. Die Druckluft weist deshalb ein Druckniveau auf, das vorzugsweise nicht über 2 bar liegt, um eine ungewollte Beschädigung der zu reinigenden englumigen Kanäle, Rohre, Leitungen und/oder dgl. sicher vermeiden zu können.

Gemäß einem weiteren Merkmal der Erfindung ist vorgesehen, dass ein dritter eingangsseitiger Anschluss ein Anschluss für Druckluft eines zweiten Druckniveaus von vorzugsweise unter 500 mbar ist. Dieser Druckluftanschluss dient insbesondere dazu, einen Dichtigkeitstest durchführen zu können, weshalb dieser Anschluss mit einem ausgangsseitigen Anschluss in strömungstechnischer Verbindung steht, an den ein Dichtigkeitstester strömungstechnisch angeschlossen ist.

Gemäß einem weiteren Merkmal der Erfindung ist vorgesehen, dass ein vierter eingangsseitiger Anschluss ein Anschluss für Druckluft eines dritten Druckniveaus von vorzugsweise zwischen 3 bar und 8 bar ist. Dieser Druckluftanschluss steht insbesondere mit einem der ausgangsseitigen Anschlüsse in strömungstechnischer Verbindung sowie mit dem Arbeitszylinder. Dies erlaubt es, den einen ausgangsseitigen Anschluss wahlweise mit Reinigungsflüssigkeit oder mit Druckluft auf einem vergleichsweise hohen Druckniveau, d.h. mit einem Druckniveau oberhalb von 2 mbar beschicken zu können. Andererseits dient dieser Druckluftanschluss dazu, den Arbeitszylinder druckzubeaufschlagen und damit verfahren zu können.

Gemäß einem weiteren Merkmal der Erfindung ist ein ausgangsseitiger Anschluss vorgesehen, dem ein Ventil zur wahlweisen Verbindung mit dem ersten oder dem zweiten eingangsseitigen Anschluss strömungstechnisch vorgeschaltet ist. Ein solcher ausgangsseitiger Anschluss kann wahlweise mit Reinigungsflüssigkeit oder mit Druckluft von vorzugsweise unter 2 bar beschickt.

Gemäß einem weiteren Merkmal der Erfindung ist vorgesehen, dass ein ausgangsseitiger Anschluss vorgesehen ist, dem ein Ventil zur wahlweisen Verbindung mit dem zweiten oder dem vierten eingangsseitigen Anschluss strömungstechnisch vorgeschaltet ist. Dieser ausgangsseitige Anschluss kann wahlweise mit Reinigungsflüssigkeit oder mit Druckluft von vorzugsweise zwischen 3 bar und 5 bar beschickt werden.

Gemäß einem weiteren Merkmal der Erfindung ist ein ausgangsseitiger Anschluss vorgesehen, der mit dem dritten eingangsseitigen Anschluss in strömungstechnischer Verbindung steht. Dieser ausgangsseitige Anschluss kann mithin im Bedarfsfall zu Testzwecken mit Druckluft von vorzugsweise unter 500 mbar beschickt werden.

Gemäß einem weiteren Merkmal der Erfindung ist vorgesehen, dass ein Gehäuse des Arbeitszylinders außenseitig Sensoren zur Positionserkennung der Kolbenstange trägt. Bei diesen Sensoren kann es sich beispielsweise um Magnetschalter handeln. Die Positionserkennung für die Kolbenstange ermöglicht es insbesondere, zu ermitteln, ob zur Ankopplung des Zugankers an eine Anschlusseinrichtung ein Adapterelement insbesondere in Form einer Adapterplatte Verwendung findet.

Weitere Merkmale und Vorteile von Ausführungsbeispielen der Erfindung ergeben sich aus der nachfolgenden Beschreibung anhand der Figuren. Dabei zeigen
- Fig. 1: in schematischer Darstellung eine erfindungsgemäße Reinigungseinrichtung;
- Fig. 2: in schematischer Schaltdarstellung ein erfindungsgemäßes Modul;
- Fig. 3: in schematisch perspektivischer Darstellung ein erfindungsgemäßes Modul gemäß einer ersten Ausführungsform;
- Fig. 4: in schematisch perspektivischer Darstellung das erfindungsgemäße Modul nach Fig. 3;
- Fig. 5: in schematisch perspektivischer Darstellung das erfindungsgemäß Modul nach den Figuren 3 und 4;
- Fig. 6: in schematisch perspektivischer Darstellung einen Trägerflansch eines erfindungsgemäßen Moduls gemäß einer zweiten Ausführungsform;
- Fig. 7: in schematisch perspektivischer Darstellung in rückwärtiger Ansicht den Trägerflansch nach Fig. 6;
- Fig. 8: in schematisch perspektivischer Darstellung das erfindungsgemäße Modul gemäß einer zweiten Ausführungsform;
- Fig. 9: in schematisch perspektivischer Darstellung das erfindungsgemäße Modul nach Fig. 8; und
- Fig. 10: in schematischer Schnittdarstellung das erfindungsgemäße Modul nach den Figuren 8 und 9.

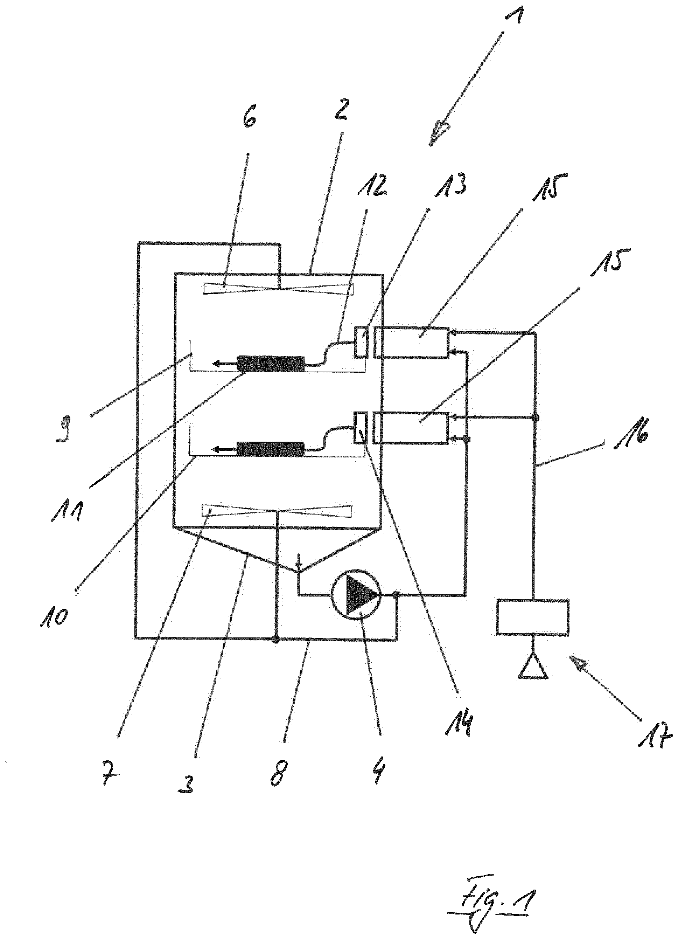

Fig. 1 lässt in schematischer Darstellung ein erfindungsgemäßes Reinigungsgerät 1 erkennen, bei dem es sich im gezeigten Ausführungsbeispiel um einen Reinigungs- und/oder Desinfektionsautomaten handelt.

Die Reinigungseinrichtung 1 verfügt über einen Spülbehälter 2, der zur Aufnahme von zu reinigendem Spülgut 11 einen Spülraum bereitstellt.

Innerhalb des Spülraums sind verfahrbar Spülgutträger 9 und 10 angeordnet, bei denen es sich im gezeigten Ausführungsbeispiel jeweils um Spülkörbe handelt. Ein jeder Spülgutträger 9 bzw. 10 stellt jeweils eine Anschlusseinrichtung 13 bzw. 14 bereit, an die ein zu reinigender englumiger Kanal 12 eines Spülguts 11 angeschlossen ist.

Innerhalb des Spülbehälters 2 ist eine Sprüheinrichtung zur Außenbeaufschlagung des Spülguts 11 mit Reinigungsflüssigkeit, der sogenannten Spülflotte, angeordnet, wobei die Sprüheinrichtung im gezeigten Ausführungsbeispiel über zwei sich drehende Sprüharme 6 und 7 verfügt.

Der Spülbehälter 2 mündet in einen Sammeltopf 3 ein. Dieser ist wiederum strömungstechnisch an eine Umwälzpumpe 4 angeschlossen, die über eine Leitung 8 in strömungstechnischer Verbindung mit den Sprüharmen 6 und 7 der Sprüheinrichtung steht. Im bestimmungsgemäßen Verwendungsfall wird über die Sprüharme 6 und 7 Spülflotte zur Außenreinigung des Spülguts 11 abgegeben, die sich anschließend im Sammeltopf 3 ansammelt und von dort aus mittels der Umwälzpumpe 4 erneut zu den Sprüharmen 6 und 7 geführt und somit im Kreislauf umgewälzt wird. Zur Innenreinigung des Spülguts 11 und insbesondere der englumigen Kanäle 12 ist die Umwälzpumpe 4 zudem an die Anschlusseinrichtungen 13 und 14 strömungstechnisch angeschlossen, und zwar unter jeweiliger Zwischenschaltung eines erfindungsgemäßen Moduls 15.

Die Module 15 sind ferner über eine Druckluftleitung 16 an eine Druckluftquelle 17 angeschlossen. Es ist so ermöglicht, die Module 15 sowohl mit Spülflotte als auch mit Druckluft zu beschicken, was ein Hindurchführen durch die englumigen Kanäle 12 des Spülguts 11 entweder von Reinigungsflüssigkeit oder von Druckluft ermöglicht.

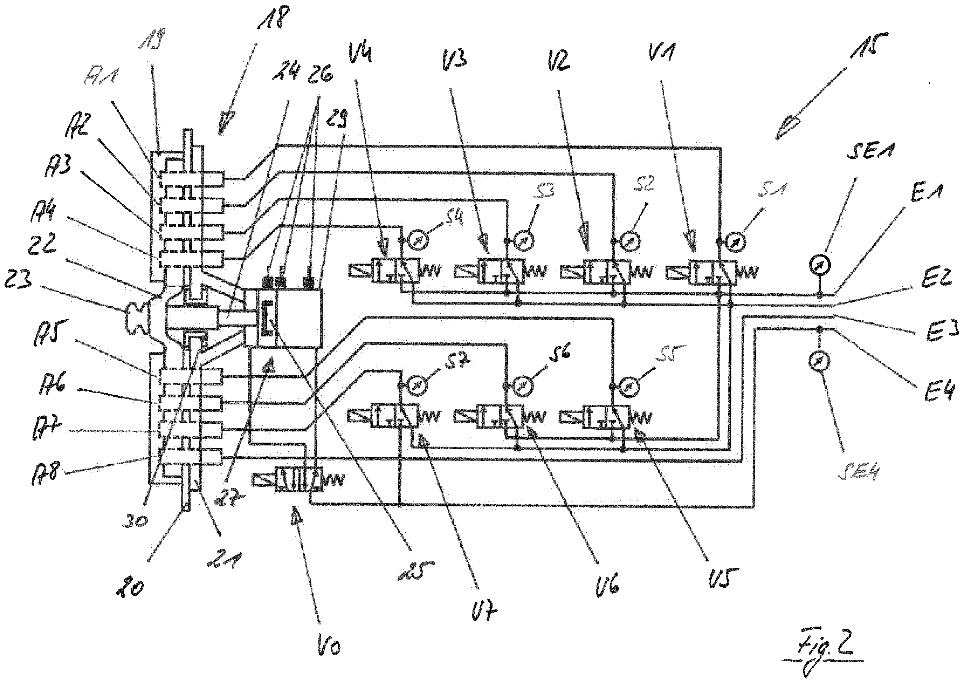

Der Aufbau eines erfindungsgemäßen Moduls 15 ist anhand eines schematischen Schaltplans in Fig. 2 dargestellt.

Wie Fig. 2 erkennen lässt, verfügt ein Modul 15 über eine Mediumverteileinrichtung, die eine Vielzahl von eingangsseitigen Anschlüssen E1, E2, E3 und E4 sowie eine Vielzahl von ausgangsseitigen Anschlüssen A1, A2, A3, A4, A5, A6, A7 und A8 aufweist. Dabei handelt es sich bei dem ersten eingangsseitigen Anschluss E1 um einen Anschluss für Spülflotte. Die weiteren eingangsseitigen Anschlüsse E2, E3 und E4 sind jeweils ein Anschluss für Druckluft. Dabei stellt der zweite eingangsseitige Anschluss E2 einen Anschluss für Druckluft eines ersten Druckniveaus von vorzugweise unter 2 bar, der dritte eingangsseitige Anschluss E3 einen Anschluss für Druckluft eines zweiten Druckniveaus von vorzugsweise unter 500 mbar und der vierte eingangsseitige Anschluss E4 einen Anschluss für Druckluft eines dritten Druckniveaus von vorzugsweise zwischen 3 bar und 5 bar bereit. Einem jeden eingangsseitigen Anschluss E1 bis E4 kann ein Eingangsdrucksensor SE1 bis SE4 zugeordnet sein.

Zur wahlweisen Beschickung der ausgangsseitigen Anschlüsse A1 bis A7 mit Reinigungsflüssigkeit und/oder Druckluft sind Ventile V1 bis V7 vorgesehen. Dabei sind den ausgangsseitigen Anschlüssen A1 bis A6 die Ventile V1 bis V6 strömungstechnisch vorgeschaltet, so dass die ausgangsseitigen Anschlüsse A1 bis A6 jeweils wahlweise über den eingangsseitigen Anschluss E1 mit Reinigungsflüssigkeit oder über den zweiten eingangsseitigen Anschluss E2 mit Druckluft mit einem Druckniveau von vorzugsweise unter 2 bar beaufschlagt werden können. An jeden dieser ausgangsseitigen Anschlüsse A1 bis A6 kann im bestimmungsgemäßen Verwendungsfall der englumige Kanal 12 eines Spülguts 11 strömungstechnisch angeschlossen sein.

Dem ausgangsseitigen Anschluss A7 ist das Ventil V7 vorgeschaltet. Dieses steht in strömungstechnischer Verbindung mit den eingangsseitigen Anschlüssen E2 und E4. Es ist mithin eine wahlweise Beschickung des ausgangsseitigen Anschlusses A7 mit Druckluft möglich, und zwar auf einem Druckniveau von vorzugsweise unter 2 bar einerseits sowie auf einem Druckniveau vorzugsweise zwischen 3 bar und 5 bar andererseits.

Der ausgangsseitige Anschluss A8 ist ohne Zwischenschaltung eines Ventils direkt an den eingangsseitigen Anschluss E3 angeschlossen, was eine Beschickung dieses ausgangsseitigen Anschlusses mit Druckluft auf einem Druckniveau von vorzugsweise unter 500 mbar ermöglicht.

Den Ventilen V1 bis V7 ist jeweils ein Drucksensor S1 bis S7 nachgeschaltet.

Für ein Ankoppeln eines Moduls 15 an eine spülgutträgerseitige Anschlusseinrichtung 13 bzw. 14 verfügt ein Modul 15 über einen Arbeitszylinder 27. Dieser verfügt über einen in einem Gehäuse 29 geführten Kolben 25. Der Kolben 25 ist an eine Kolbenstange 24 angeschlossen, die endseitig einen Anker 23 trägt.

Im gezeigten Ausführungsbeispiel ist der Arbeitszylinder 27 als doppelwirkender Druckzylinder ausgebildet und unter Zwischenordnung des Ventils V0 an den vierten eingangsseitigen Anschluss E4 angeschlossen. Es ist so eine Druckbeaufschlagung des Arbeitszylinders 24 mit Druckluft auf einem Druckniveau vorzugsweise zwischen 3 bar und 5 bar ermöglicht.

Außen am Gehäuse 29 des Arbeitszylinders 27 sind im gezeigten Ausführungsbeispiel drei Magnetschalter 26 angeordnet, die eine Positionsermittlung des Kolbens gestatten. Hierüber lässt sich erkennen, ob eine optionale Adapterplatte am Anker 23 angeschlossen ist.

Zur Anordnung eines Moduls 15 am Spülbehälter 2 einer Reinigungseinrichtung 1 ist ein Trägerflansch 18 vorgesehen. Dieser verfügt über eine Anschlussplatte 19 einerseits und eine Widerlagerplatte 21 andererseits, die im endmontierten Zustand die Wand 20 des Spülbehälters 2 zwischen sich aufnehmen. Zur Abdichtung gegenüber dem vom Spülbehälter 2 bereitgestellten Spülraum ist eine Dichtung 22 vorgesehen. Zudem stellt der Trägerflansch 18 die Lagerung 30 für die längsverschiebliche Anordnung der Kolbenstange 24 des Arbeitszylinders 27 bereit.

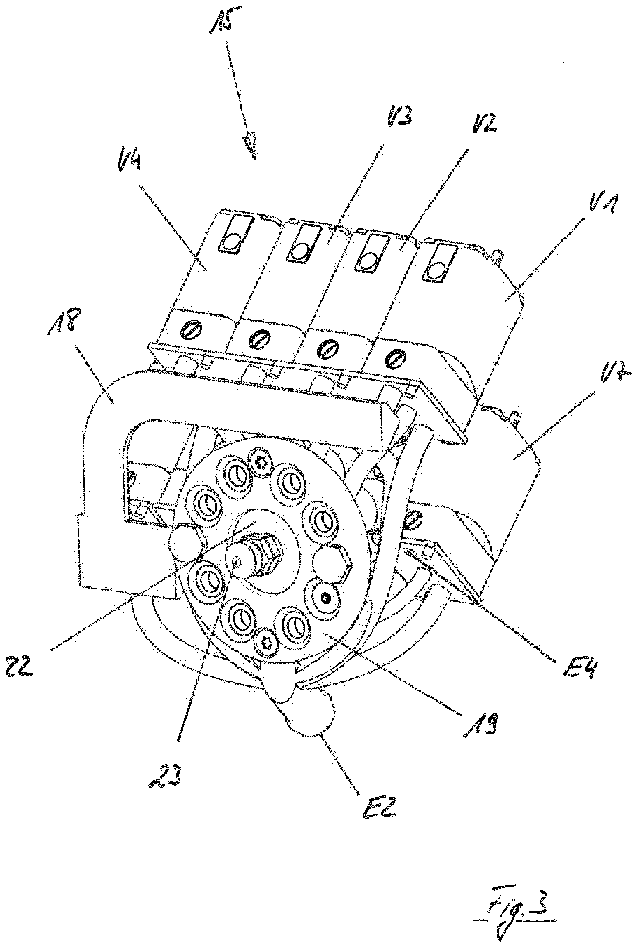

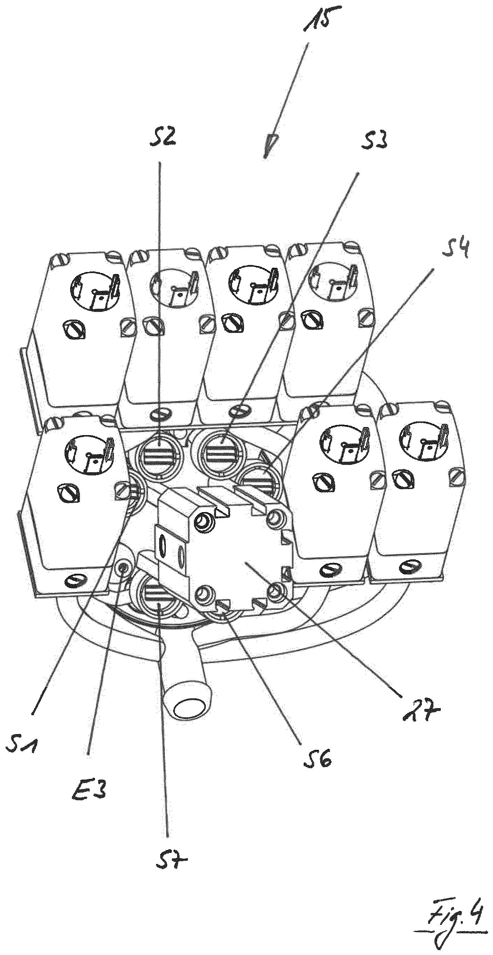

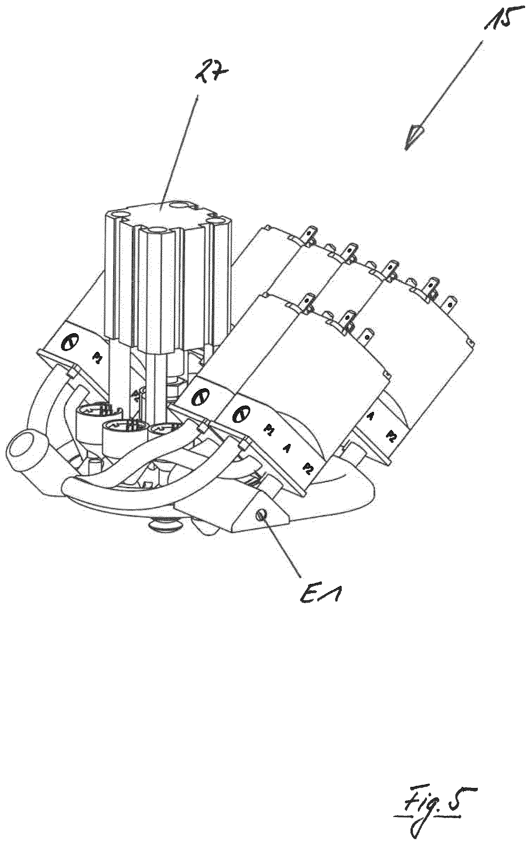

Die Figuren 3 bis 5 zeigen in jeweils unterschiedlicher Ansicht in jeweils perspektivischer Darstellung ein erfindungsgemäßes Modul 15 gemäß einer ersten Ausführungsform. Es sind gemäß dieser Darstellungen insbesondere die Anordnung der Ventile V1 bis V7 sowie die Anordnung des Arbeitszylinders 27 zu erkennen.

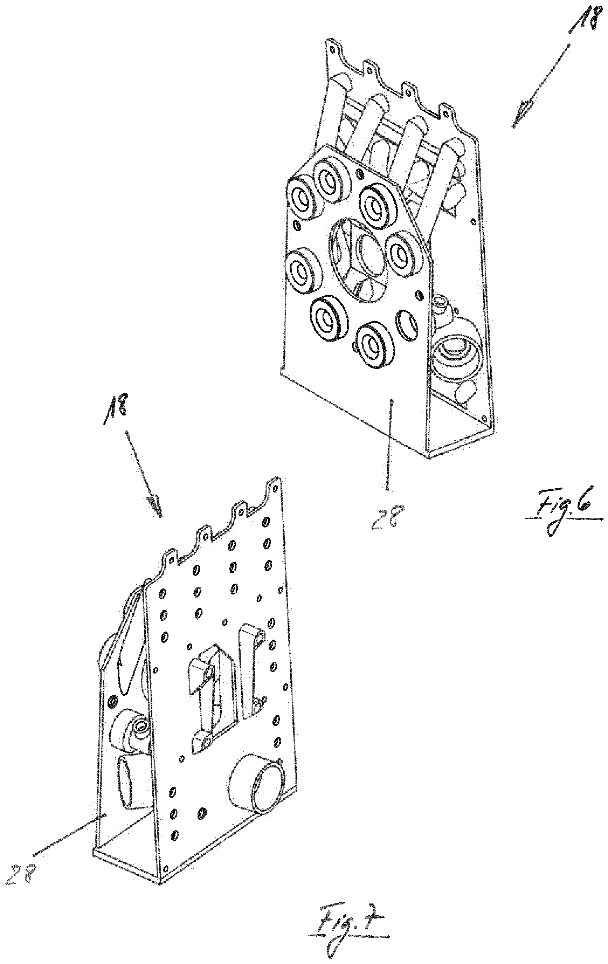

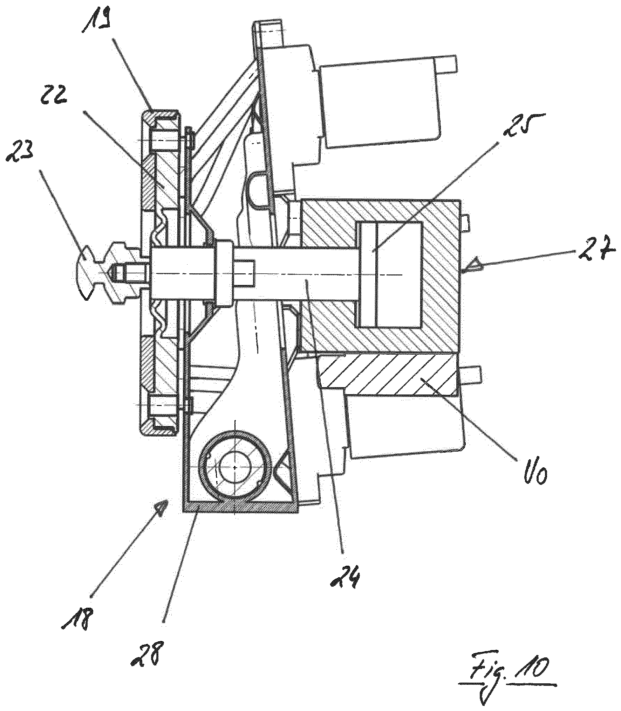

Die Figuren 6 bis 10 zeigen eine zweite Ausführungsform eines erfindungsgemäßen Moduls 15. Diese Ausführungsform unterscheidet sich von der Ausführungsform gemäß der Figuren 3 bis 5 insbesondere in der Ausgestaltung des Trägerflanschs 18. Die Funktionsweise entspricht aber der vorerläuterten.

Der Trägerflansch kann in beiden gezeigten Ausführungsformen im 3D-Druckverfahren hergestellt sein, wobei speziell ein selektives Laserschmelzen von Metall, insbesondere Edelstahl, bevorzugt ist.

Das erfindungsgemäße Modul 15 zeichnet sich insbesondere durch seine Ankoppelfunktion mittels pneumatischem Zylinder aus. Es ist so insgesamt ein kompakter Aufbau gegeben, wobei das Modul 15 einerseits eine Ankopplung an eine zugehörige Anschlusseinrichtung 13 bzw. 14 ermöglicht und zudem andererseits eine wahlweise Beaufschlagung der ausgangsseitigen Anschlüsse A1 bis A8 mit Reinigungsflüssigkeit und/oder Druckluft über die eingangsseitigen Anschlüsse E1 bis E4 gewährleistet.

Über die außenseitig am Gehäuse 29 des Arbeitszylinders 27 angebrachten Magnetschalter 26 lassen sich die beiden Endlagen der Kolbenstange 24 detektieren, so dass zwischen einem abgekoppelten Zustand und einem angekoppelten Zustand des Moduls 15 unterschieden werden kann. Ferner lässt sich mittels eines dritten Magnetschalters 26 erkennen, ob eine Adapterplatte am Anker 23 angeschlossen ist. Dabei ist dieser dritte Magnetschalter an der Stelle am Zylinder angebracht, an dem die Adapterplatte über den Anker im Normalfall in die Dichtung gezogen wird.

Hinsichtlich des Arbeitszylinders 27 kann in vorteilharter Weise auf konventionelle hooksche Federelemente wie Zug- und Druckfedern verzichtet werden, da eine einheitliche, vom Verfahrweg unabhängige Kraft aufgrund des definierten Eingangsdrucks über den eingangsseitigen Anschluss E4 und der Kolben- bzw. Kolbenringfläche des Arbeitszylinders 27 realisiert wird.

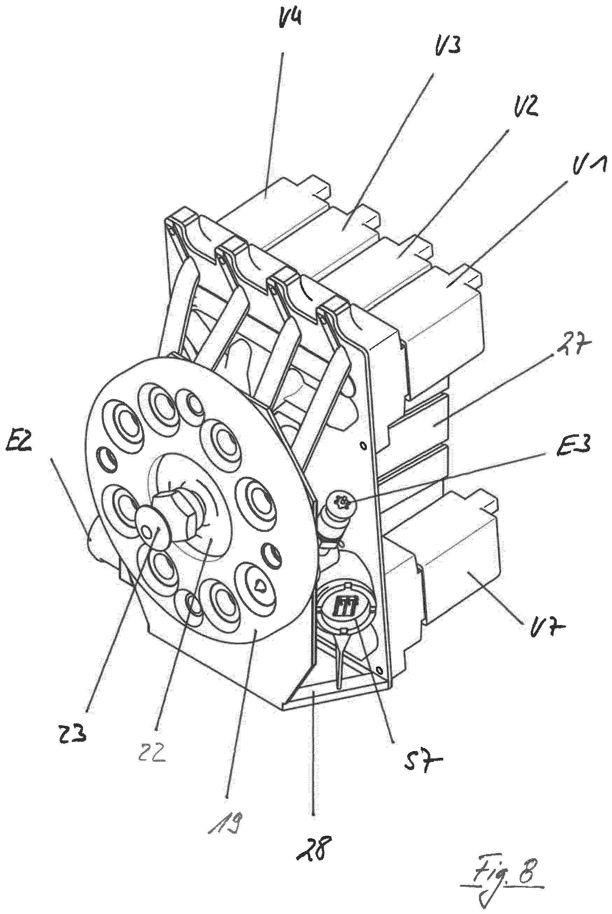

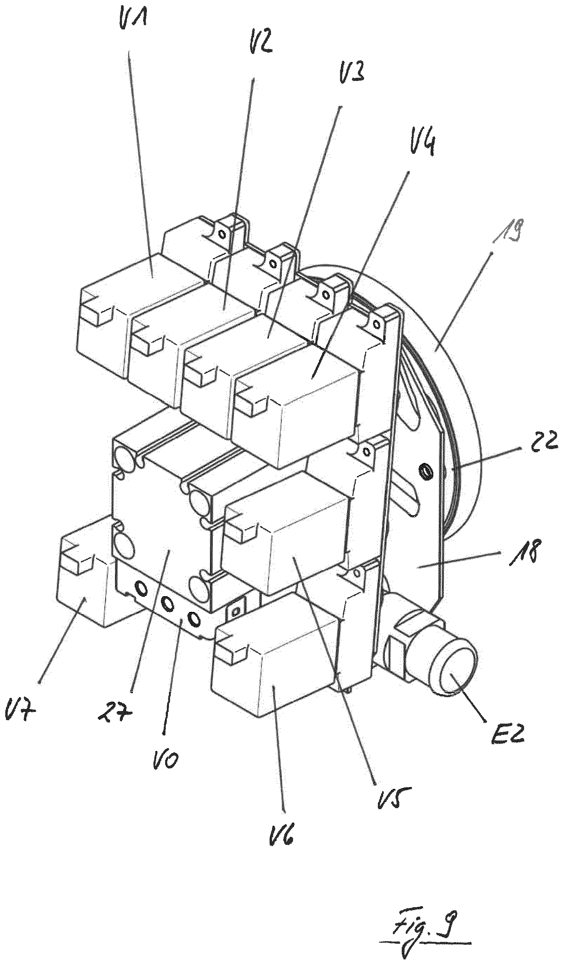

Gemäß der Ausführungsform nach den Figuren 6 bis 10 kommt ein im 3D-Druckverfahren hergestellter Trägerflansch 18 zum Einsatz, der über einen Grundkörper 28 verfügt. An diesem sind im endmontierten Zustand die Ventile V0 bis V7 sowie der Arbeitszylinder 27 angeordnet, wie dies die Figuren 8 und 9 erkennen lassen. Es ist so ein insgesamt einfacher Aufbau gegeben.

Aufgrund der Funktionskombination von Mediumverteileinrichtung und Ankoppelmechanismus an eine spülgutträgerseitige Anschlusseinrichtung ergibt sich nicht nur ein insgesamt robuster und verkleinerter Aufbau, es werden im Übrigen folgende Merkmale erreicht:
- geringere Wassermenge für Aufbereitungsprozesse notwendig
- geringere Energiemenge für Aufheizen der Wassermenge erforderlich
- kürzere Aufheizphasen durch geringen Energiebedarf
- schnellere Wasserwechselvorgänge durch geringere Wassermengen pro Spülschritt
- schnellere Aufbereitungsprozesse durch kürzere Aufheizphasen und Wasserwechselvorgänge und
- Reduktion der Montagezeit durch weniger Komponenten und Verschlauchungen.

### Bezugszeichen

- 1: Reinigungseinrichtung
- 2: Spülbehälter
- 3: Sammeltopf
- 4: Umwälzpumpe
- 6: Sprüharm
- 7: Sprüharm
- 8: Leitung
- 9: Spülgutträger
- 10: Spülgutträger
- 11: Spülgut
- 12: Kanal
- 13: Anschlusseinrichtung
- 14: Anschlusseinrichtung
- 15: Modul
- 16: Druckluftleitung
- 17: Druckluftquelle
- 18: Trägerflansch
- 19: Anschlussplatte
- 20: Spülbehälterwand
- 21: Widerlagerplatte
- 22: Dichtung
- 23: Anker
- 24: Kolbenstange
- 25: Kolben
- 26: Magnetschalter
- 27: Arbeitszylinder
- 28: Grundkörper
- 29: Gehäuse
- 30: Lagerung

- E1 bis E4: eingangsseitiger Anschluss
- A1 bis A8: ausgangsseitiger Anschluss
- SE1 bis SE4: Sensor eingangsseitiger Anschluss E1 bis E4
- S1 bis S7: Drucksensor
- V0 bis V7: Ventil

## Patentansprüche

1. Modul zur Beaufschlagung von in einem Spülbehälter (2) einer Reinigungseinrichtung (1) angeordneten englumigen Kanälen, Leitungen, Rohren und/oder dgl. von Spülgütern (11), insbesondere Endoskopen, mit einem Medium, mit einer Mediumverteileinrichtung, die eine Vielzahl von eingangsseitigen Anschlüssen (E1 - E4) und ausgangsseitigen Anschlüssen (A1 - A8) aufweist, wobei die eingangsseitigen Anschlüsse (E1 - E4) mit den ausgangsseitigen Anschlüssen (A1 - A8) in strömungstechnischer Verbindung stehen, **gekennzeichnet durch** einen Trägerflansch (18), der einen Arbeitszylinder (27) aufweist, wobei der Arbeitszylinder (27) in strömungstechnischer Verbindung mit einem eingangsseitigen Anschluss (E1 - E4) steht, und wobei der Arbeitszylinder (27) eine Kolbenstange (24) bereitstellt, die endseitig einen mit einer Anschlusseinrichtung (13, 14) eines Spülgutträgers (9, 10) zusammenwirkenden Anker (23) aufweist.

2. Modul nach Anspruch 1,
**dadurch gekennzeichnet, dass**
der Arbeitszylinder (27) ein pneumatischer Zylinder ist.

3. Modul nach Anspruch 2,
**dadurch gekennzeichnet, dass**
der pneumatische Zylinder ein Druckzylinder ist.

4. Modul nach Anspruch 3,
**dadurch gekennzeichnet, dass**
der Druckzylinder ein doppelwirkender Druckzylinder ist.

5. Modul nach einem der vorhergehenden Ansprüche,
**dadurch gekennzeichnet, dass**
ein erster eingangsseitiger Anschluss (E1) ein Anschluss für eine Reinigungsflüssigkeit ist.

6. Modul nach einem der vorhergehenden Ansprüche,
**dadurch gekennzeichnet, dass**
ein zweiter eingangsseitiger Anschluss (E2) ein Anschluss für Druckluft eines ersten Druckniveaus von vorzugsweise unter 2 bar ist.

7. Modul nach einem der vorhergehenden Ansprüche,
**dadurch gekennzeichnet, dass**
ein dritter eingangsseitiger Anschluss (E3) ein Anschluss für Druckluft eines zweiten Druckniveaus von vorzugsweise unter 500 mbar ist.

8. Modul nach einem der vorhergehenden Ansprüche,
**dadurch gekennzeichnet, dass**
ein vierter eingangsseitiger Anschluss (E4) ein Anschluss für Druckluft eines dritten Druckniveaus von vorzugsweise zwischen 3 bar und 5 bar ist.

9. Modul nach einem der vorhergehenden Ansprüche,
**dadurch gekennzeichnet, dass**
der Arbeitszylinder (27) mit dem vierten eingangsseitigen Anschluss (E4) in strömungstechnischer Verbindung steht.

10. Modul nach einem der vorhergehenden Ansprüche,
**dadurch gekennzeichnet, dass**
ein Gehäuse (29) des Arbeitszylinders (27) außenseitig Sensoren zur Positionserkennung der Kolbenstange (24) trägt.

11. Modul nach Anspruch 10,
**dadurch gekennzeichnet, dass**
die Sensoren Magnetschalter sind.

12. Modul nach einem der vorhergehenden Ansprüche,
**dadurch gekennzeichnet, dass**
ein ausgangsseitiger Anschluss (A1 - A6) vorgesehen ist, dem ein Ventil (V1 bis V6) zur wahlweisen Verbindung mit dem ersten oder dem zweiten eingangsseitigen Anschluss (E1, E2) strömungstechnisch vorgeschaltet ist.

13. Modul nach einem der vorhergehenden Ansprüche,
**dadurch gekennzeichnet, dass**
ein ausgangsseitiger Anschluss (A7) vorgesehen ist, dem ein Ventil (V7) zur wahlweisen Verbindung mit dem zweiten oder dem vierten eingangsseitigen Anschluss (E2, E4) strömungstechnisch vorgeschaltet ist.

14. Modul nach einem der vorhergehenden Ansprüche,
**dadurch gekennzeichnet, dass**
ein ausgangsseitiger Anschluss (A8) vorgesehen ist, der mit dem dritten eingangsseitigen Anschluss (E3) in strömungstechnischer Verbindung steht.

15. Reinigungseinrichtung, insbesondere Reinigungs- und/oder Desinfektionsautomat, mit einem einen Spülraum bereitstellenden Spülbehälter (2), der der Aufnahme von zu reinigendem Spülgut (11) dient, mit einem Modul (15) nach einem der vorhergehenden Ansprüche 1 bis 14.

## Claims

1. Module for applying a medium to narrow-necked channels, lines, pipes and/or the like of washware (11), in particular endoscopes, arranged in a washing container (2) of a cleaning device (1), the module comprising a medium distribution device which has a plurality of inlet-side connections (E1 - E4) and outlet-side connections (A1 - A8), wherein the inlet-side connections (E1 - E4) are fluidically connected to the outlet-side connections (A1 - A8), **characterised by** a carrier flange (18), which has a working cylinder (27), wherein the working cylinder (27) is fluidically connected to an inlet-side connection (E1 - E4), and wherein the working cylinder (27) provides a piston rod (24) which has an armature (23) at its end, which armature interacts with a connection device (13, 14) of a washware carrier (9, 10).

2. Module according to claim 1,
**characterised in that**
the working cylinder (27) is a pneumatic cylinder.

3. Module according to claim 2,
**characterised in that**
the pneumatic cylinder is a pressure cylinder.

4. Module according to claim 3,
**characterised in that**
the pressure cylinder is a double-acting pressure cylinder.

5. Module according to any of the preceding claims,
**characterised in that**
a first inlet-side connection (E1) is a connection for a cleaning fluid.

6. Module according to any of the preceding claims,
**characterised in that**
a second inlet-side connection (E2) is a connection for compressed air of a first pressure level of preferably less than 2 bar.

7. Module according to any of the preceding claims,
**characterised in that**
a third inlet-side connection (E3) is a connection for compressed air of a second pressure level of preferably less than 500 mbar.

8. Module according to any of the preceding claims,
**characterised in that**
a fourth inlet-side connection (E4) is a connection for compressed air of a third pressure level of preferably between 3 bar and 5 bar.

9. Module according to any of the preceding claims,
**characterised in that**
the working cylinder (27) is fluidically connected to the fourth inlet-side connection (E4).

10. Module according to any of the preceding claims,
**characterised in that**
a housing (29) of the working cylinder (27) has sensors on the outside for detecting the position of the piston rod (24).

11. Module according to claim 10,
**characterised in that**
the sensors are magnetic switches.

12. Module according to any of the preceding claims,
**characterised in that**
an outlet-side connection (A1 - A6) is provided, upstream of which a valve (V1 to V6) for optional connection to the first or the second inlet-side connection (E1, E2) is fluidically connected.

13. Module according to any of the preceding claims,
**characterised in that**
an outlet-side connection (A7) is provided, upstream of which a valve (V7) for optional connection to the second or the fourth inlet-side connection (E2, E4) is fluidically connected.

14. Module according to any of the preceding claims,
**characterised in that**
an outlet-side connection (A8) is provided which is fluidically connected to the third inlet-side connection (E3).

15. Cleaning device, in particular a cleaning and/or disinfection machine, comprising a washing container (2) which provides a washing chamber and receives washware (11), and comprising a module (15) according to any of the preceding claims 1 to 14.

## Revendications

1. Module permettant l'alimentation de canaux, conduites, tubes et/ou autres objets similaires à lumière étroite, lesquels sont disposés dans une cuve de lavage (2) d'un dispositif de nettoyage (1), de produits à laver (11), en particulier d'endoscopes, en fluide, comportant un dispositif de distribution de fluide qui présente une pluralité de raccords côté entrée (E1 - E4) et de raccords côté sortie (A1 - A8), dans lequel les raccords côté entrée (E1 - E4) sont en liaison selon la technique des fluides avec les raccords côté sortie (A1 - A8), **caractérisé par** une bride de support (18) qui présente un cylindre de travail (27), dans lequel le cylindre de travail (27) est en liaison selon la technique des fluides avec un raccord côté entrée (E1 - E4), et dans lequel le cylindre de travail (27) fournit une tige de piston (24) qui présente côté extrémité une ancre (23) coopérant avec un dispositif de raccordement (13, 14) d'un support pour produit à laver (9, 10).

2. Module selon la revendication 1,
**caractérisé en ce que**
le cylindre de travail (27) est un cylindre pneumatique.

3. Module selon la revendication 2,
**caractérisé en ce que**
le cylindre pneumatique est un cylindre de pression.

4. Module selon la revendication 3,
**caractérisé en ce que**
le cylindre de pression est un cylindre de pression à double effet.

5. Module selon l'une des revendications précédentes,
**caractérisé en ce**
**qu'**un premier raccord côté entrée (E1) est un raccord pour un liquide de nettoyage.

6. Module selon l'une des revendications précédentes,
**caractérisé en ce**
**qu'**un deuxième raccord côté entrée (E2) est un raccord pour de l'air comprimé d'un premier niveau de pression de préférence inférieur à 2 bar.

7. Module selon l'une des revendications précédentes,
**caractérisé en ce**
**qu'**un troisième raccord côté entrée (E3) est un raccord pour de l'air comprimé d'un deuxième niveau de pression de préférence inférieur à 500 mbar.

8. Module selon l'une des revendications précédentes,
**caractérisé en ce**
**qu'**un quatrième raccord côté entrée (E4) est un raccord pour de l'air comprimé d'un troisième niveau de pression de préférence compris entre 3 bar et 5 bar.

9. Module selon l'une des revendications précédentes,
**caractérisé en ce que**
le cylindre de travail (27) est en liaison selon la technique des fluides avec le quatrième raccord côté entrée (E4).

10. Module selon l'une des revendications précédentes,
**caractérisé en ce**
**qu'**un boîtier (29) du cylindre de travail (27) supporte côté externe des capteurs pour la détection de position de la tige de piston (24).

11. Module selon la revendication 10,
**caractérisé en ce que**
les capteurs sont des commutateurs magnétiques.

12. Module selon l'une des revendications précédentes,
**caractérisé en ce**
**qu'**un raccord côté sortie (A1 - A6) est prévu, en amont duquel est montée une soupape (V1 à V6) pour la liaison au choix avec le premier ou le deuxième raccord côté entrée (E1, E2) selon la technique des fluides.

13. Module selon l'une des revendications précédentes,
**caractérisé en ce**
**qu'**un raccord côté sortie (A7) est prévu, en amont duquel est montée une soupape (V7) pour la liaison au choix avec le deuxième ou le quatrième raccord côté entrée (E2, E4) selon la technique des fluides.

14. Module selon l'une des revendications précédentes,
**caractérisé en ce**
**qu'**un raccord côté sortie (A8) est prévu, qui est en liaison selon la technique des fluides avec le troisième raccord côté entrée (E3).

15. Dispositif de nettoyage, en particulier automate de nettoyage et/ou de désinfection, comportant une cuve de lavage (2) fournissant un espace de lavage, laquelle cuve de lavage sert à la réception de produits à laver (11), comportant un module (15) selon l'une des revendications précédentes 1 à 14.
